# EUROPEAN PATENT APPLICATION

(11) **EP 3 323 339 A1**
(43) Date of publication of application: **23.05.2018**
(21) Application number: 16824404.4
(22) Date of filing: 08.07.2016
(51) Int. Cl.: A61B 1/04

(54) **IMAGE DISPLAY CONTROL DEVICE, DISPLAY DEVICE AND IMAGE DISPLAY SYSTEM**

(30) Priority: 16.07.2015 JP 2015142071
(71) Applicant: Kimura, Masashi, Nishinomiya-shi, Hyogo 669-1103 (JP)
(72) Inventor: Kimura, Masashi, Nishinomiya-shi, Hyogo 669-1103 (JP)
(74) Representative: Viering, Jentschura & Partner mbB Patent- und Rechtsanwälte
(86) International application number: PCT/JP2016/070285
(87) International publication number: WO 2017/010424

(57) **Abstract**

An image display control device (100) displays a human body interior image on a display device (200) and includes an imaging section (1), a transmission section (2), an image acquiring section (31), a transmitter (32), and a power source (33). The imaging section (1) images the interior of a human body and generates human body interior image data. The image acquiring section (31) acquires the human body interior image data corresponding to the human body interior image from the imaging section (1) through the transmission section (2). The transmitter (32) transmits the human body interior image data acquired by the image acquiring section (31) to the display device (200) through wireless communication. The power source (33) supplies power to the imaging section (1), the image acquiring section (31), and the transmitter (32). The image acquiring section (31), the transmitter (32), and the power source (33) are integral to one another, forming a main unit (3).

## Description

### [TECHNICAL FIELD]

The present invention relates to an image display control device for displaying an image of the interior of a human body on a display device. The present invention also relates to a display device and an image display system.

### [BACKGROUND ART]

Image display control devices for displaying an image of the interior of a human body on a display device are commonly known. As an example of such image display control devices, a probe-type observation device is disclosed (see Patent Literature 1).

The probe-type observation device disclosed in Patent Literature 1 includes a probe body, magnifying optics, a high magnification imaging means, an image display section, and an image recording section. The probe body is inserted into an endoscope for observing a subject. The magnifying optics is disposed on a tip of the probe body and magnifies an image by a greater magnification than observation optics of the endoscope. The high magnification imaging means captures an image of a subject through the magnifying optics. The image display section displays the image captured by the high magnification imaging means. The image recording section records the captured image displayed on the image display section. The image display section and the image recording section are provided in a portable housing that is portable and connectable to a base end of the probe body.

Patent Literature 1 describes that the probe-type observation device allows a user to perform observation while holding the portable housing, requires no installation space, and is carriageable and usable when needed.

### [CITATION LIST]

### [Patent Literature]

[Patent Literature 1]
Japanese Patent Application Laid-Open Publication No. 2005-13484

### [SUMMARY OF INVENTION]

### [Technical Problem]

However, the probe body of the probe-type observation device disclosed in Patent Literature 1 is connected to a light source with a cable, and the cable may interfere with movement of a physician who is moving while holding the probe body. The light source supplies, to an irradiation section, light for irradiating the subject. Another problem is that, for example, it is difficult to display an image easily viewable to a patient at a position easily viewable to the patient when the physician explains to the patient about an endoscopic image.

The present invention was achieved in consideration of the above problems and an objective thereof is to provide an image display control device, a display device, and an image display system that do not interfere with a user's action and that allow a patient or the like to easily view an image of the interior of a human body.

### [Solution to Problem]

A first image display control device according the present invention displays a human body interior image on a display device and includes an image acquiring section, a transmitter, and a power source. The image acquiring section acquires human body interior image data corresponding to the human body interior image. The transmitter transmits the human body interior image data acquired by the image acquiring section to the display device through wireless communication. The power source supplies power to the image acquiring section and the transmitter. The image acquiring section, the transmitter, and the power source are integral to one another, forming a main unit of the image display control device.

A second image display control device according to the present invention is the first image display control device further including an imaging section and a transmission section. The imaging section images the interior of a human body and generates the human body interior image data. The transmission section transmits the human body interior image data generated by the imaging section to the image acquiring section.

A third image display control device according to the present invention is the second image display control device in which the imaging section is integral to the transmission section. The transmission section is attachable to and detachable from the main unit.

A fourth image display control device according to the present invention is the second image display control device or the third image display control device in which the power source supplies power to the imaging section.

A fifth image display control device according to the present invention is any one of the second to fourth image display control devices in which the transmitter is configured to be communicable with a plurality of the display devices using a different frequency for each of the display devices.

A sixth image display control device according to the present invention is the fifth image display control device in which intensity of radio waves to be output by the transmitter is determined such that each of the display devices displays the human body interior image corresponding to the human body interior image data when a distance between the display device and the image display control device is equal to or smaller than a predetermined specific distance.

A first display device according to the present invention receives and displays human body interior image data from any one of the second to sixth image display control devices. The first display device includes a receiver and a display section. The receiver receives the human body interior image data from the image display control device. The display section displays a human body interior image corresponding to the human body interior image data received by the receiver.

A second display device according to the present invention is the first display device in which the display section displays the human body interior image at a location on a line connecting a position of a pupil of a user and a position of the interior of the human body.

A third display device according to the present invention is the second display device in which the display section includes a screen and a projector. The screen is positioned on the line connecting the position of the pupil of the user and the position of the interior of the human body. The projector projects, onto the screen, the human body interior image corresponding to the human body interior image data received by the receiver.

A fourth display device according to the present invention is the third display device according to the present invention in which the screen is a drape for covering a patient.

A fifth display device according to the present invention is the first display device in which the display section displays the human body interior image on an upper section of at least one of right and left lenses of a pair of eyeglasses.

A sixth display device according to the present invention is the fifth display device further comprising an operation receiver and a transparency setting section. The operation receiver receives input from a user of an operation of setting transparency of the human body interior image. The transparency setting section sets the transparency of the human body interior image based on the input of the operation received by the operation receiver. The display section displays the human body interior image on at least one of the right and left lenses with the transparency set by the transparency setting section.

A seventh display device according to the present invention is any one of the first to sixth display devices further comprising a recording section. The recording section records the human body interior image data.

An image display system according to the present invention includes any one of the first to sixth image display control devices and any one of the first to seventh display devices.

### [Advantageous Effects of Invention]

The image display control device, the display device, and the image display system according to the present invention do not interfere with a user's action and allow a patient or the like to easily view an image of the interior of a human body.

### [BRIEF DESCRIPTION OF DRAWINGS]

[FIG. 1]

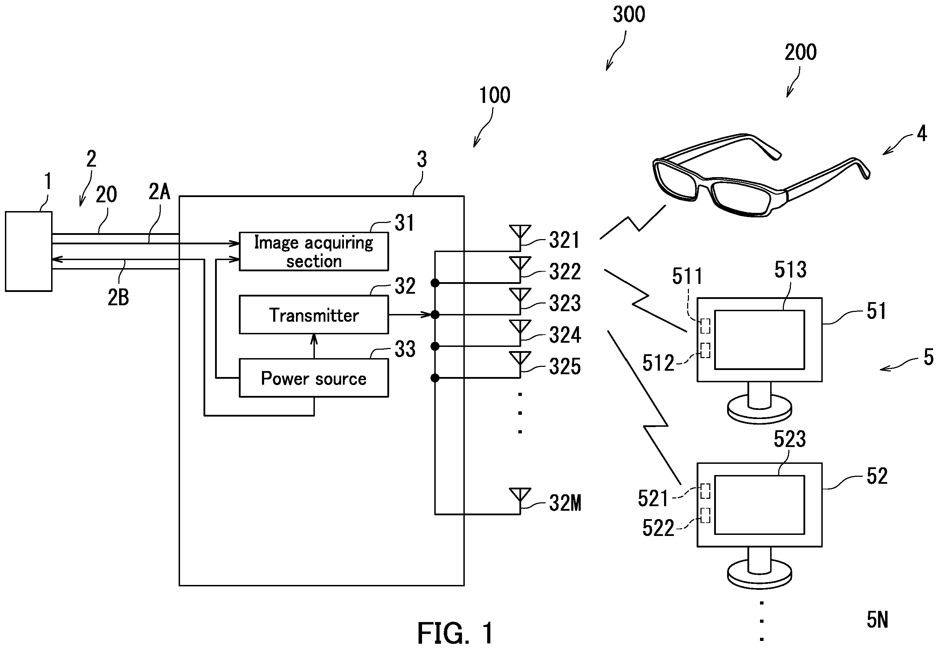

   FIG. 1 is a diagram illustrating a configuration of an image display system according to an embodiment of the present invention.
[FIG. 2]

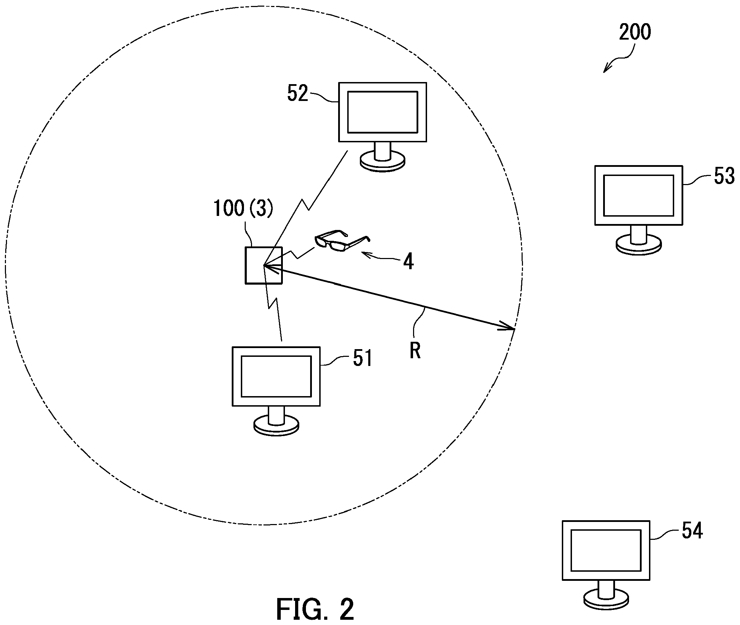

   FIG. 2 is a diagram illustrating a communicable range of an image display control device illustrated in FIG. 1.
[FIG. 3]

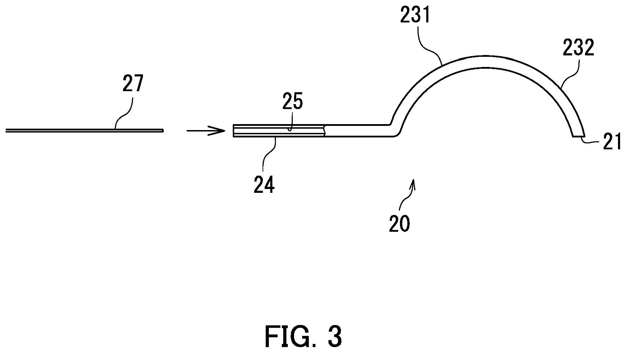

   FIG. 3 is a diagram illustrating a configuration of a guide tube illustrated in FIG. 1.
[FIG. 4]

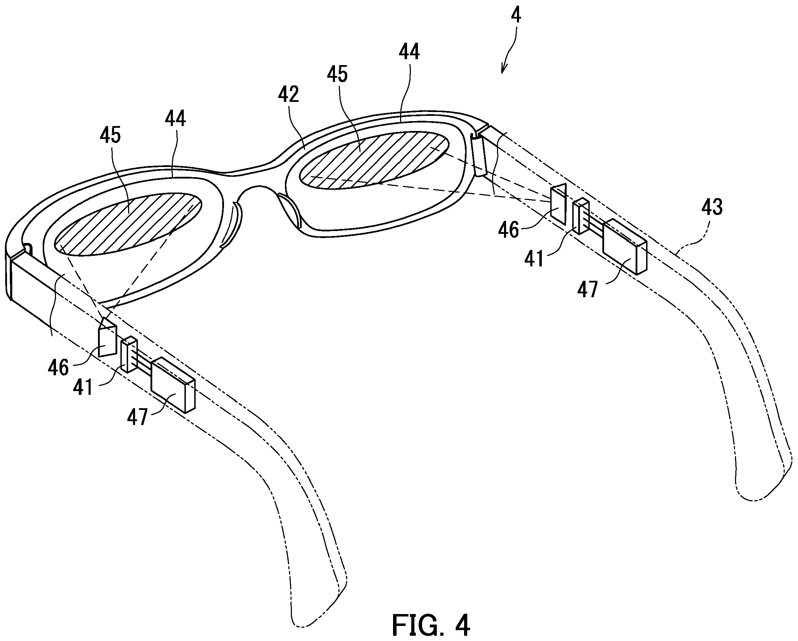

   FIG. 4 is a perspective view illustrating a configuration of a display device illustrated in FIG. 2.
[FIG. 5]

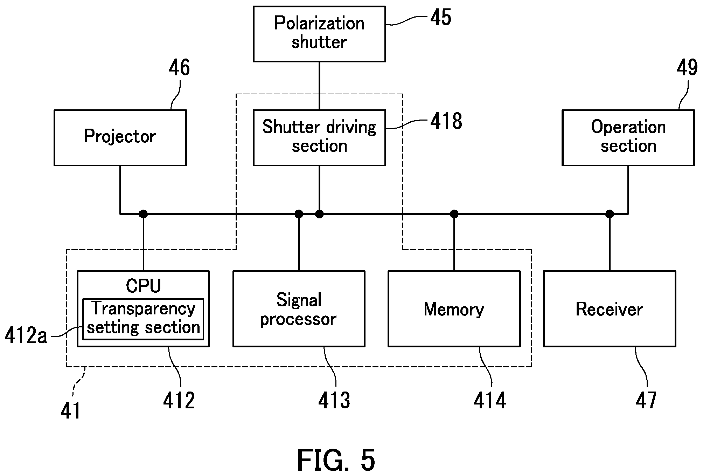

   FIG. 5 is a block diagram illustrating the configuration of the display device illustrated in FIG. 4.
[FIG. 6]

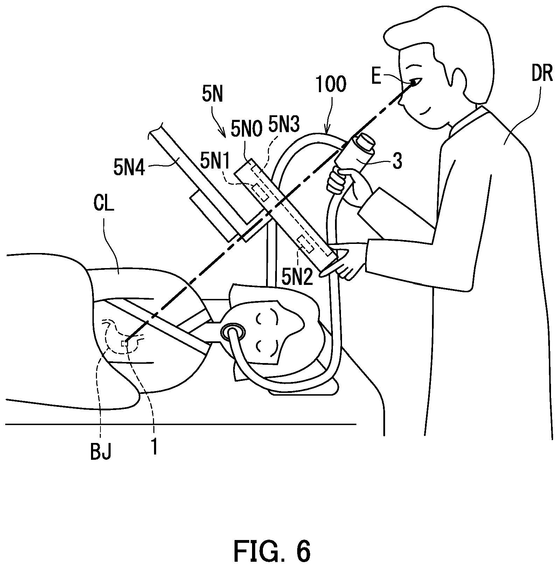

   FIG. 6 is a diagram illustrating an example of display devices illustrated in FIG. 2.

### [DESCRIPTION OF EMBODIMENTS]

The following describes an embodiment of the present invention with reference to the drawings (FIGS. 1 to 6). Elements that are the same or equivalent are indicated by the same reference signs in the drawings and description thereof is not repeated.

First, an image display system 300 according to the present embodiment will be described with reference to FIG. 1. FIG. 1 is a diagram illustrating a configuration of the image display system 300 according to the embodiment of the present invention. The image display system 300 includes an image display control device 100 and display devices 200. The image display control device 100 includes an imaging section 1, a transmission section 2, and a main unit 3. The main unit 3 includes an image acquiring section 31, a transmitter 32, and a power source 33.

The imaging section 1 images the interior of a human body and generates human body interior image data. Specifically, the imaging section 1 includes a light emitting diode (LED) and a complementary metal-oxide-semiconductor (CMOS) image sensor. The LED irradiates a subject (for example, pharynx) inside a human body. The CMOS image sensor generates image data of the subject inside the human body.

The transmission section 2 includes a data transmission wire 2A, a power supply wire 2B, and a guide tube 20. The transmission section 2 transmits, to the main unit 3 (image acquiring section 31), the human body interior image data generated by the imaging section 1. The power supply wire 2B supplies power from the main unit 3 (power source 33) to the imaging section 1. The data transmission wire 2A and the power supply wire 2B are each formed of a coaxial cable, for example. The guide tube 20 has a hollow cylindrical shape, and the data transmission wire 2A and the power supply wire 2B are disposed in the interior of the guide tube 20. The imaging section 1 is integral to the transmission section 2. One end (right end in FIG. 1) of the transmission section 2 is attachable to and detachable from the main unit 3.

The main unit 3 includes a central processing unit (CPU), read only memory (ROM), and random access memory (RAM). The CPU functions as the image acquiring section 31, the transmitter 32, and the power source 33 through execution of control programs stored in the ROM.

The image acquiring section 31 acquires the human body interior image data generated by the imaging section 1 through the transmission section 2. The image acquiring section 31 also converts the acquired human body interior image data into image data in a format displayable by the display devices 200.

The transmitter 32 transmits the human body interior image data that has been acquired and converted by the image acquiring section 31 to each of the display devices 200 through wireless communication. The transmitter 32 is capable of communication using a plurality of (for example, 253) channels (transmit frequencies). Examples of transmit frequencies that can be used by the transmitter 32 include a 2.4 GHz band. In FIG. 1, antennae 321 and 322 through 32M represent different transmit frequencies used by the transmitter 32.

The power source 33 supplies power to the image acquiring section 31 and the transmitter 32. The power source 33 also supplies power to the imaging section 1 through the transmission section 2 (power supply wire 2B). The power source 33 for example includes a rechargeable battery or a dry-cell battery.

The display device 200 receives the human body interior image data from the image display control device 100 and displays a human body interior image corresponding to the human body interior image data in a manner viewable to a user. The display device 200 for example includes a display device 4 of eyewear type and display devices 5 each having an liquid crystal display (LCD). The display device 4 will be described below with reference to FIGS. 4 and 5.

The display devices 5 include display devices 51 and 52 through 5N. The display devices 51 and 52 through 5N have substantially the same configuration as one another, and therefore the configuration of the display device 51 will be described for convenience. The display device 51 includes a receiver 511, a display controller 512, and a display section 513. Likewise, the display device 52 includes a receiver 521, a display controller 522, and a display section 523. The display device 51 and the display device 52 have substantially the same configuration, and therefore the configuration of the display device 51 will be described for convenience. The receiver 511 receives the human body interior image data from the image display control device 100. Specifically, the receiver 511 receives radio waves with a frequency allocated to the display device 51 by the transmitter 32 of the image display control device 100. The display controller 512 generates the human body interior image based on the human body interior image data received by the receiver 511 and causes the display section 513 to display the image. The display section 513 includes an LCD and the like and displays the human body interior image.

Since the image display control device 100 transmits the human body interior image data to each of the display devices 200 through wireless communication as described above, the image display control device 100 does not interfere with the user's action, and a patient or the like can easily view the human body interior image. In other words, since the human body interior image data is transmitted to each of the display devices 200 through wireless communication, there is no cable that may interfere with movement of a physician who is moving while carrying the image display control device 100, for example.

The image display system 300 includes the image display control device 100 and the display devices 200. Since the image display control device 100 transmits the human body interior image data to each of the display devices 200 through wireless communication, the image display control device 100 does not interfere with the user's action, and a patient or the like can easily view the human body interior image. In other words, since the human body interior image data is transmitted to each of the display devices 200 through wireless communication, there is no cable that may interfere with movement of a physician who is moving while carrying the image display control device 100, for example.

Furthermore, since the human body interior image data is transmitted to each of the plurality of display devices 200, the same human body interior image data can be displayed on a plurality of display devices such as the display device 4 that displays the image in a manner viewable to a physician, the display device 51 that is placed in a position easily viewable to a patient, and the display device 52 that is placed in a position easily viewable to other people (observers, students, and the like). As a position easily viewable to a patient lying on the back, for example, the display device 51 can be placed in a position above the head of the patient.

Since the image display control device 100 includes the imaging section 1 and the transmission section 2, a user, such as a physician, can capture the human body interior image data while carrying the image display control device 100.

Furthermore, since the imaging section 1 is integral to the transmission section 2 and the transmission section 2 is attachable to and detachable from the main unit 3, the imaging section 1 and the transmission section 2 can be disposable. The imaging section 1 and the transmission section 2 that are disposable do not need to be sterilized, increasing usability.

Furthermore, since the main unit 3 (power source 33) supplies power to the imaging section 1, the image display control device 100 does not need to have an additional power source for supplying power to the imaging section 1. Thus, the image display control device 100 can have a simplified configuration.

The present embodiment is described as an embodiment in which the display devices 200 directly receive the human body interior image data wirelessly transmitted from the image display control device 100. According to another embodiment, however, the display devices 200 may receive the human body interior image data wirelessly transmitted by the image display control device 100 via a public wireless network. According to such an embodiment, the image display control device 100 can transmit the human body interior image data to the display devices 200 in remote locations.

The present embodiment is described as an embodiment in which the imaging section 1 includes the LED and the CMOS image sensor. According to another embodiment, however, the imaging section 1 may include a charge coupled device (CCD) image sensor instead of the CMOS image sensor.

Preferably, Wi-Fi Video Transmitter VT-100 (product name), product of Scalar Corporation can for example be used as the image display control device 100.

The following describes a communicable range, in which the image display control device 100 (main unit 3) is communicable with the display devices 200, with reference to FIG. 2. FIG. 2 is a diagram illustrating the communicable range of the image display control device 100 illustrated in FIG. 1. The display devices 200 include a display device 53 and a display device 54 in addition to the display device 4, the display device 51, and the display device 52 illustrated in FIG. 1. The display device 53 and the display device 54 have substantially the same configuration as the display device 51.

The intensity of radio waves to be output by the transmitter 32 (see FIG. 1) is determined such that each of the display devices 200 displays the human body interior image corresponding to the human body interior image data when a distance L between the display device 200 and the image display control device 100 is equal to or smaller than a predetermined specific distance R (for example, five meters).

In other words, the communicable range in which the image display control device 100 is communicable with the display devices 200 is the specific distance R (five meters in this example). Accordingly, the display device 4, the display device 51, and the display device 52 that are within a radius of the specific distance R from the image display control device 100 display the human body interior image, but the display device 53 and the display device 54 that are outside the radius of the specific distance R from the image display control device 100 do not display the human body interior image.

A desired communicable range can be set by appropriately determining the intensity of the radio waves to be output by the transmitter 32 (see FIG. 1) as described above. In a situation in which an oral cavity endoscopy is performed, for example, only a display device 200 located in a room where the endoscopy is performed is enabled to display the human body interior image corresponding to the human body interior image data from the image display control device 100.

In other words, the display devices 200 located outside the room where the endoscopy is performed are not enabled to display the human body interior image corresponding to the human body interior image data from the image display control device 100, and thus leakage of the human body interior image data can be prevented.

The following describes a configuration of the guide tube 20 with reference to FIG. 3. FIG. 3 is a diagram illustrating the configuration of the guide tube 20 illustrated in FIG. 1. In order to provide a passage for inserting and withdrawing a transnasal endoscope, the guide tube 20 according to the present embodiment has an initial shape designed based on a nasal cavity shape. The guide tube 20 can for example be made from silicone, polyethylene, or vinyl chloride.

More specifically, the guide tube 20 has the initial shape having a straight section 24, a first curved section 231, and a second curved section 232. The straight section 24 connects to the first curved section 231, and the first curved section 231 connects to the second curved section 232. The first curved section 231 extends from an end of the straight section 24 in a direction away from an axis 241 of the straight section 24. The second curved section 232 extends from an end of the first curved section 231 in a direction toward the axis 241 of the straight section 24.

The initial shape of the guide tube 20 matches the shape of a part from the nasal cavity to the entrance of the pharyngeal cavity. The guide tube 20 deforms upon receiving external force but is able to recover the initial shape upon removal of the external force. It is therefore possible to deform the guide tube 20 into a shape that matches the shape of the nasal cavity by controlling external force on the guide tube 20 while inserting the guide tube 20 into the nasal cavity and then into the entrance of the pharyngeal cavity. As a result, the guide tube 20 can be inserted into the nasal cavity appropriately.

Specifically, the guide tube 20 can be inserted into the nasal cavity from a nostril with a first end 21 of the guide tube 20 directed diagonally upward, and subsequently advanced into the pharyngeal cavity with the first end 21 of the guide tube 20 directed downward. As a result, an in-tube space 25 of the guide tube 20 provides a passage for inserting and withdrawing the transnasal endoscope. A tip of an insertion section of the transnasal endoscope can reach the vicinity of the pharyngeal cavity through the in-tube space 25. Although the above description uses the transnasal endoscope as an object to be inserted or withdrawn through the in-tube space 25 of the guide tube 20, the present invention is not limited to the description. The object may be a different object (for example, air, a drug, or a syringe).

The guide tube 20 can be straightened by inserting a bar 27 into the in-tube space 25 of the guide tube 20 and thus giving external force to the guide tube 20. The bar 27 is for example a plastic bar.

The following describes the display device 4 with reference to FIGS. 4 and 5. FIG. 4 is a perspective view illustrating a configuration of the display device 4 illustrated in FIG. 2. The display device 4 is a head mounted display (HMD) of eyewear type. A physician, for example, wears the display device 4 and captures a human body interior image using the image display control device 100. The display device 4 includes controllers 41, a lens frame 42, ear stems 43, lenses 44, polarization shutters 45, projectors 46, and receivers 47.

The lens frame 42 and the ear stems 43 form an eyewear frame. The pair of lenses 44 is attached to the lens frame 42. The polarization shutters 45 are pasted on upper sections of the pair of lenses 44. The polarization shutters 45 are each formed of a liquid crystal display element and are each switchable between an open state for transmitting light and a closed state for blocking light. Switching between the open state and the closed state is achieved by controlling voltage that is applied to a liquid crystal layer of the liquid crystal display element.

The receivers 47 receive the human body interior image data from the image display control device 100 (see FIG. 1). The controllers 41 generate the human body interior image corresponding to the human body interior image data received by the receivers 47 and transmit the generated human body interior image to the projectors 46. The controllers 41 also open and close the polarization shutters 45. The projectors 46 project the human body interior image input from the controllers 41 onto the polarization shutters 45.

Since the polarization shutters 45 are pasted on the upper sections of the respective lenses 44 as described above, the human body interior image is displayed on the upper sections of the lenses 44. Thus, a physician wearing the display device 4, for example, can view the human body interior image as necessary while maintaining a visual field needed for carrying out a treatment or test (referred to as an actional visual field) through lower sections of the lenses 44. Thus, working efficiency of the user such as a physician is increased.

The present embodiment is described as an embodiment in which the polarization shutters 45 are pasted on the upper sections of the pair of lenses 44. According to another embodiment, however, a polarization shutter 45 may be pasted only on the upper section of one (for example, right one) of the lenses 44. Thus, the user, such as a physician, can maintain a visual field needed for carrying out a treatment or test through the left lens 44 and the right lens 44. Such a configuration provides the physician wearing the display device 4 with a greater actional visual field.

The following describes the display device 4 with reference to FIG. 5. The display device 4 further includes an operation section 49. The controllers 41 each include a CPU 412, a signal processor 413, memory 414, and a shutter driving section 418. The controllers 41, the projectors 46, the receivers 47, and the operation section 49 are communicatively connected to one another through a bus.

The signal processors 413 perform signal processing on the human body interior image data received by the receivers 47 and generate the human body interior image corresponding to the human body interior image data. A control program, the human body interior image data, various set values, and the like are stored in the memory 414. The shutter driving sections 418 drive the polarization shutters 45 in accordance with an instruction from the CPUs 412.

The operation section 49 is disposed in an appropriate position on the display device 4 and receives input of the user's operation. For example, the operation section 49 receives input of operations of opening and closing the polarization shutters 45. The operation section 49 corresponds to an example of an "operation receiver".

The CPUs 412 control operation of the display device 4. The CPUs 412 each function as a transparency setting section 412a through execution of the control program stored in the memory 414.

The transparency setting sections 412a set transparency of the human body interior image based on the input of the operation received by the operation section 49. Specifically, the transparency setting sections 412a set the polarization shutters 45 to the open state or the closed state based on the input of the operation received by the operation section 49. For example, upon the operation section 49 receiving input of an operation of opening the polarization shutters 45, the transparency setting sections 412a instruct the shutter driving sections 418 to open the polarization shutters 45. Upon the operation section 49 receiving input of an operation of closing the polarization shutters 45, the transparency setting sections 412a instruct the shutter driving sections 418 to close the polarization shutters 45.

When the polarization shutters 45 are in the open state, light is transmitted, and thus the human body interior image from the projectors 46 is not displayed. In other words, the transparency of the human body interior image is 100%. When the polarization shutters 45 are in the closed state, light is blocked, and thus the human body interior image from the projectors 46 is displayed. In other words, the transparency of the human body interior image is 0%.

The polarization shutters 45 are opened or closed through operation of the operation section 49, thereby changing transparency of the human body interior image. Thus, a physician wearing the display device 4, for example, can perform a treatment or test on a patient while viewing the human body interior image by setting the transparency to 0% (by closing the polarization shutters 45). The physician wearing the display device 4 can also perform the treatment or test on the patient without his/her view blocked by the human body interior image by setting the transparency to 100% (by opening the polarization shutters 45). The user, such as the physician, wearing the display device 4 can therefore readily switch the human body interior image between being displayed and not being displayed. Thus, usability can be further improved.

The present embodiment is described as an embodiment in which the transparency of the human body interior image is set to 0% or 100%. According to another embodiment, however, the transparency of the human body interior image may be set to any value in a range of from 0% to 100%. According to such an embodiment, the user can display the human body interior image with a desired transparency. Thus, usability can be further improved.

The following describes an example (display device 5N) of the display devices 5 with reference to FIG. 6. FIG. 6 is a diagram illustrating the example (display device 5N) of the display devices 5 illustrated in FIG. 2. The display device 5N includes a receiver 5N1, a display controller 5N2, and a display section 5N3. The receiver 5N1 receives the human body interior image data from the image display control device 100. Specifically, the receiver 5N1 receives radio waves with a frequency allocated to the display device 5N by the transmitter 32 of the image display control device 100. The display controller 5N2 generates the human body interior image from the human body interior image data received by the receiver 5N1 and causes the display section 5N3 to display the image. The display section 5N3 is for example formed of an LCD and displays the human body interior image.

The display device 5N further includes a housing 5N0 and a support 5N4. The housing 5N0 houses the receiver 5N1, the display controller 5N2, and the display section 5N3. The support 5N4 supports the housing 5N0. The support 5N4 has multiple joints and supports the housing 5N0 while positioning and fixing the housing 5N0 in a position and at an angle desired by a user (for example, a physician DR) by receiving external force from the user.

In FIG. 6, the image display control device 100 is a gastroscope. The physician DR operates the image display control device 100. In FIG. 6, an observation region of a stomach BJ of a patient CL is the human body interior. In other words, the physician DR operates the image display control device 100 (gastroscope) to position the imaging section 1 at the observation region of the stomach BJ of the patient CL. The physician DR also positions the display device 5N on a line (a dashed line in FIG. 6) connecting a position of a pupil E of the physician DR and a position of the observation region (human body interior) of the stomach BJ of the patient CL.

Since the display device 5N is positioned on the line connecting the position of the pupil E of the physician DR and the position of the observation region (human body interior) of the stomach BJ of the patient CL, an "observational visual field" of the physician DR can match a "procedural visual field" of the physician DR. The "procedural visual field" is a visual field available when the physician DR is viewing a procedure area. In FIG. 6, the procedure area for the physician DR corresponds to the position of the observation region of the stomach BJ of the patient CL. The "observational visual field" is a visual field available when the physician DR is viewing an observation target. In FIG. 6, the observation target for the physician DR is the observation region of the stomach BJ of the patient CL.

Since the "observational visual field" of the physician DR matches the "procedural visual field" of the physician DR, the physician DR can have the "observational visual field" and the "procedural visual field" at the same time without moving the location of gaze. Thus, working efficiency of the physician DR can be increased.

Referring to FIG. 6, the present embodiment is described as an embodiment in which the display device 5N is positioned on the line connecting the position of the pupil E of the physician DR and the position of the observation region (human body interior) of the stomach BJ of the patient CL. However, the present invention is not limited thereto. According to another embodiment, for example, the display device may include a screen and a projector. According to such an embodiment, the screen is positioned on the line connecting the position of the pupil E of the physician DR and the position of the observation region (human body interior) of the stomach BJ of the patient CL. The projector projects, onto the screen, the human body interior image corresponding to the human body interior image data received by the receiver. In such a configuration, for example, the screen may be a drape for covering the patient CL. In such a configuration, for example, the observation region (human body interior) of the stomach BJ of the patient CL imaged by the imaging section 1 is displayed at a location on the drape covering the patient CL that corresponds to the stomach. Thus, work efficiency of the physician DR can be further increased.

The display device 4 described with reference to FIGS. 4 and 5 is an eyewear type HMD wearable for the physician DR. The human body interior image is displayed on the lenses 44 of the display device 4. As in the example illustrated in FIG. 6, therefore, the display device 4 is positioned on the line connecting the position of the pupil E of the physician DR and the position of the observation region (human body interior) of the patient CL. As a result, the "observational visual field" of the physician DR can match the "procedural visual field" of the physician DR, and therefore the physician DR can have the "observational visual filed" and the "procedural visual field" at the same time without moving the location of gaze. Thus, working efficiency of the physician DR can be increased.

Through the above, embodiments of the present invention have been described with reference to the drawings. However, the present invention is not limited to the above embodiments and may be implemented in various different forms that do not deviate from the essence of the present invention (for example, as described below in sections (1)-(5)). The drawings schematically illustrate elements of configuration in order to facilitate understanding and properties of elements of configuration illustrated in the drawings, such as thickness, length, and number thereof, may differ from actual properties thereof in order to facilitate preparation of the drawings. Furthermore, properties of elements of configuration described in the above embodiments, such as shapes and dimensions, are merely examples and are not intended as specific limitations. Various alterations may be made so long as there is no substantial deviation from the configuration of the present invention.
(1) The present embodiment is described as an embodiment in which the human body interior image is an endoscopic image. According to another embodiment, however, the human body interior image may be a different type of image. For example, according to an embodiment, the human body interior image may be an ultrasound image or a fluoroscopic image (digital subtraction angiography: DSA).
(2) The present embodiment is described as an embodiment in which the image acquiring section 31 acquires the human body interior image data generated by the imaging section 1 through the transmission section 2. According to another embodiment, however, the human body interior image data may be acquired in a different manner. For example, according to an embodiment, the image acquiring section 31 may acquire the human body interior image data generated by the imaging section 1 through wireless communication. In such an embodiment, the transmission section 2 can be omitted, simplifying the configuration of the image display control device 100.
(3) The present embodiment is described as an embodiment in which the transmitter 32 transmits radio waves to the display devices 200 with a different frequency for each of the display devices 200. According to another embodiment, however, the transmitter 32 may transmit radio waves with a single frequency to the plurality of display devices 200. The transmitter 32 according to such an embodiment can have a simplified configuration.
(4) The present embodiment is described as an embodiment in which a physician wears the display device 4. According to another embodiment, however, a user who is not a physician may wear the display device 4. In an embodiment in which a nurse wears the display device 4, for example, the nurse can view the human body interior image as necessary while maintaining an actional visual field.
(5) The present embodiment is described as an embodiment in which the display devices 200 display the human body interior image. According to another embodiment, however, the display devices 200 may each include a recording section for recording the human body interior image data on a storage medium such as a hard disk drive (HDD). According to such an embodiment, the display devices 200 can each display the human body interior image corresponding to the human body interior image data stored in the recording section.

### [INDUSTRIAL APPLICABILITY]

The present invention is applicable to image display control devices that display human body interior images on display devices and to display devices.

### [REFERENCE SIGNS LIST]

- 300: Image display system
- 100: Image display control device
- 1: Imaging section
- 2: Transmission section
- 2A: Data transmission wire
- 2B: Power supply wire
- 20: Guide tube
- 231: First curved section
- 232: Second curved section
- 24: Straight section
- 25: In-tube space
- 27: Bar
- 3: Main unit
- 31: Image acquiring section
- 32: Transmitter
- 33: Power source
- 200: Display device
- 4: Display device
- 41: Controller
- 412: CPU
- 412a: Transparency setting section
- 414: Memory
- 418: Shutter driving section
- 42: Lens frame
- 43: Ear stem
- 44: Lens
- 45: Polarization shutter
- 46: Projector
- 47: Receiver
- 49: Operation section
- 51, 52, 53, 54, 5N: Display device
- 5N0: Housing
- 511, 521, 5N1: Receiver
- 512, 522, 5N2: Display controller
- 513, 523, 5N3: Display section
- 5N4: Support
- DR: Physician
- CL: Patient
- BJ: Stomach (example of human body interior)

## Claims

1. An image display control device for displaying a human body interior image on a display device, the image display control device comprising:
an image acquiring section configured to acquire human body interior image data corresponding to the human body interior image;
a transmitter configured to transmit the human body interior image data acquired by the image acquiring section to the display device through wireless communication; and
a power source configured to supply power to the image acquiring section and the transmitter, wherein
the image acquiring section, the transmitter, and the power source are integral to one another, forming a main unit of the image display control device.

2. The image display control device according to claim 1, further comprising:
an imaging section configured to image the interior of a human body and generate the human body interior image data; and
a transmission section configured to transmit the human body interior image data generated by the imaging section to the image acquiring section.

3. The image display control device according to claim 2, wherein
the imaging section is integral to the transmission section, and
the transmission section is attachable to and detachable from the main unit.

4. The image display control device according to claim 2 or 3, wherein
the power source supplies power to the imaging section.

5. The image display control device according to any one of claims 2 to 4, wherein
the transmitter is configured to be communicable with a plurality of the display devices using a different frequency for each of the display devices.

6. The image display control device according to claim 5, wherein
intensity of radio waves to be output by the transmitter is determined such that each of the display devices displays the human body interior image when a distance between the display device and the image display control device is equal to or smaller than a predetermined specific distance.

7. A display device for receiving human body interior image data from the image display control device according to any one of claims 2 to 6 and displaying a human body interior image corresponding to the human body interior image data, the display device comprising:
a receiver configured to receive the human body interior image data from the image display control device; and
a display section configured to display the human body interior image corresponding to the human body interior image data received by the receiver.

8. The display device according to claim 7, wherein
the display section displays the human body interior image at a location on a line connecting a position of a pupil of a user and a position of the interior of the human body.

9. The display device according to claim 8, wherein
the display section includes:
a screen configured to be positioned on the line connecting the position of the pupil of the user and the position of the interior of the human body; and
a projector configured to project, onto the screen, the human body interior image corresponding to the human body interior image data received by the receiver.

10. The display device according to claim 9, wherein
the screen is a drape for covering a patient.

11. The display device according to claim 7, wherein
the display section displays the human body interior image on an upper section of at least one of right and left lenses of a pair of eyeglasses.

12. The display device according to claim 11, further comprising:
an operation receiver configured to receive input from a user of an operation of setting transparency of the human body interior image; and
a transparency setting section configured to set the transparency of the human body interior image based on the input of the operation received by the operation receiver, wherein
the display section displays the human body interior image on at least one of the right and left lenses with the transparency set by the transparency setting section.

13. The display device according to any one of claims 7 to 12, further comprising
a recording section configured to record the human body interior image data.

14. An image display system comprising:
the image display control device according to any one of claims 1 to 6; and
the display device according to any one of claims 7 to 13.
